(19) Europäisches Patentamt / European Patent Office / Office européen des brevets

(11) **EP 1 203 068 B1**

(12) **EUROPEAN PATENT SPECIFICATION**

(45) Date of publication and mention
of the grant of the patent:
**03.09.2008 Bulletin 2008/36**

(21) Application number: **00946107.0**

(22) Date of filing: **14.07.2000**

(51) Int Cl.:
*C11D 17/00* (2006.01)    *C11D 3/22* (2006.01)
*C09K 8/20* (2006.01)    *A01N 25/30* (2006.01)

(86) International application number:
**PCT/GB2000/002725**

(87) International publication number:
**WO 2001/005932 (25.01.2001 Gazette 2001/04)**

(54) **STRUCTURED SURFACTANT SYSTEMS**

STRUKTURIERTE TENSIDSYSTEME

SYSTÈMES SURFACTANT STRUCTURÉS

(84) Designated Contracting States:
**AT BE CH CY DE DK ES FI FR GB GR IE IT LI LU MC NL PT SE**

(30) Priority: **17.07.1999 GB 9916744**
**17.08.1999 GB 9919287**
**30.10.1999 GB 9925700**
**25.11.1999 GB 9927768**

(43) Date of publication of application:
**08.05.2002 Bulletin 2002/19**

(73) Proprietor: **HUNTSMAN INTERNATIONAL LLC**
**Salt Lake City, Utah 84108 (US)**

(72) Inventor: **HAWKINS, John Kinver,**
**South Staffordshire DY7 6AG (GB)**

(74) Representative: **Lawrence, John et al**
**Barker Brettell LLP**
**138 Hagley Road**
**Edgbaston**
**Birmingham**
**B16 9PW (GB)**

(56) References cited:
**EP-A- 0 414 549**    **EP-A- 0 472 089**
**EP-A- 0 732 394**    **WO-A-00/36079**
**WO-A-01/00778**    **WO-A-91/13125**
**WO-A-98/11871**    **GB-A- 2 194 793**

**Description**

The present invention relates to the formulation of structured surfactant suspending systems. It is particularly relevant to the formulation of personal care formulations such as shampoos and skin cleansing preparations.

## STRUCTURED SURFACTANT

Suspending solids in liquids presents a problem. If the solids differ in density from the liquid they will tend either to sediment or float. Increasing the viscosity of the liquid can retard, but not prevent such separation, and high viscosities are generally undesirable. Colloidal systems, in which the suspended particles are sufficiently small to experience Brownian motion, e.g. less than 1 micron, may be kinetically stable. However the difficulty or undesirability of comminuting some solids to such sizes, and the impossibility of maintaining many of them at this level in the face of crystal growth or agglomeration, limits the use of colloidal suspensions.

Adjusting the density of one phase to match that of the other is usually impracticable. Moreover such systems are almost always temperature-unstable due to differential rates of thermal expansion.

One method of suspension which permits even relatively large particles to be stably suspended is structured surfactant. The term covers systems in which a surfactant mesophase, usually a lamellar or G-phase, alone or more usually interspersed with an aqueous phase, provides a yield stress which is sufficient, when the system is at rest, to immobilise any suspended particles, but which is sufficiently low to allow the system to be poured like a normal liquid. Such systems may display very low apparent viscosities when stirred, pumped or poured and yet be capable of maintaining particles, sometimes of millimetre or larger size, indefinitely in suspension.

Three main types of suspending system have been employed in practice, all involving a G-phase, in which bilayers of surfactant are arranged with the hydrophobic part of the molecule on the interior and the hydrophilic part on the exterior of the bilayer (or vice versa). The bilayers lie side by side, e.g. in a parallel or concentric configuration, sometimes separated by aqueous layers. G-phases (also known as $L_{\alpha}$ phases) can usually be identified by their characteristic textures under the polarising microscope and/or by x-ray diffraction, which is often able to detect evidence of lamellar symmetry. Such evidence may comprise first, second and sometimes third order peaks with d-spacing ( $\dfrac{2\Pi}{Q}$ where Q is the momentum transfer vector) in a simple integral ratio 1:2:3. Other types of symmetry give different ratios, usually non-integral.

The d-spacing of the first peak in the series corresponds to the repeat spacing of the bilayer system.

Most surfactants form a G-phase either at ambient or at some higher temperature when mixed with water in certain specific proportions. However such conventional G-phases do not usually function as structured suspending systems. Useful quantities of solid render them unpourable and smaller amounts tend to sediment.

The main types of structured system used in practice are based on dispersed lamellar, spherulitic and expanded lamellar phases. Dispersed lamellar phases are two phase systems in which the surfactant bilayers are arranged as parallel plates to form domains of G-phases which are interspersed with an aqueous phase to form an opaque gel-like system. They are described in EP O 086 614.

Spherulitic phases comprise well defined spheroidal bodies, usually referred to in the art as spherulites, in which surfactant bilayers are arranged as concentric shells. The spherulites usually have a diameter in the range 0.1 to 15 microns and are dispersed in an aqueous phase in the manner of a classical emulsion, but interacting to form a structured system. Spherulitic systems are described in more detail in EP O 151 884.

Many structured surfactant systems are intermediate between dispersed lamellar and spherulitic, involving both types of structure. Usually systems having a more spherulitic character are preferred because they tend to have lower viscosity. A variant on the spherulitic system comprises prolate or rod shaped bodies sometimes referred to as batonettes.

Both of the foregoing systems comprise two phases. Their stability depends on the presence of sufficient dispersed phase to pack the system so that the interaction between the spherulites or other dispersed mesophase domains prevents separation. If the amount of dispersed phase is insufficient, e.g. because there is not enough surfactant or because the surfactant is too soluble in the aqueous phase to form sufficient of a mesophase, the system will undergo separation and cannot be used to suspend solids. Such unstable systems are not considered to be "structured" for the purpose of this specification.

A third type of structured surfactant system comprises an expanded G-phase. It differs from the other two types of structured system in being essentially a single phase, and from conventional G-phase in having a wider d-spacing. Conventional G-phases, which typically contain 60 to 75% by weight surfactant, have a d-spacing of about 4 to 7 nanometers. Attempts to suspend solids in such phases results in stiff pastes which are either non-pourable, unstable or both. Expanded G-phases with d-spacing greater than 8, e.g. 10 to 15 nanometers, form when the electrolyte is added to aqueous surfactants at concentrations just below those required to form a normal G-phase, particularly to surfactants in the M phase. The M phase comprises surfactant molecules arranged to form cylindrical rods of indefinite length. It exhibits hexagonal symmetry and a distinctive texture under the polarising microscope. Typical M phases have so high a viscosity that they appear to be curdy solids. M phases near the lower concentration limit (the $L_1$/M phase boundary) may be pourable but have a very high viscosity and often a mucous-like appearance. Such systems tend to form expanded G-phases particularly readily on addition of sufficient electrolyte.

Expanded G-phases are described in more detail in EP O 530 708. In the absence of suspended matter they are translucent, unlike dispersed lamellar or spherulitic phases which are necessarily opaque. They are optically anisotropic and have shear dependent viscosity. In this they differ from $L_1$, phases which are micellar solutions and which include microemulsions. $L_1$, phases are clear, optically isotropic and are usually substantially Newtonian. They are unstructured and cannot suspend solids.

Some $L_1$, phases exhibit small angle x-ray diffraction spectra which show evidence of hexagonal symmetry and/or exhibit shear dependent viscosity. Such phases usually have concentrations near the $L_1$/M phase boundary and may form expanded G-phases on addition of electrolyte. However in the absence of any such addition of electrolyte they lack the yield point required to provide suspending properties, and are therefore not considered to be "structured systems" for the purpose of this specification.

Expanded G phases are usually less robust than spherulitic systems. They are liable to undergo a phase change at elevated temperatures to the optically-isotropic, unstructured $L_2$ phase. Relatively low yield stress may limit the maximum size of particle that can be stably suspended.

Most structured surfactants require the presence of electrolyte as well as surfactant and water in order to form structured systems capable of suspending solids. However certain relatively hydrophobic surfactants such as isopropylamine alkyl benzene sulphonate can form spherulites in water in the absence of electrolyte. Such surfactants are capable of suspending solids in the absence of electrolyte as described in EP O 414 549.

## APPLICATION

Structured surfactants have been applied to the problems of suspending: water insoluble or sparingly soluble builders in laundry detergent; antifoams and enzymes in laundry detergents and other surfactant systems: abrasives in hard surface cleaners: pesticides and oils in agrochemical preparations (EP O 388 239 and EP O 498 231); rock cuttings in drilling muds (EP O 430 602); dyestuffs in dyebath concentrates and printing inks (EP O 472 089); talcs. oils and other cosmetic ingredients in personal care formulations (EP O 530 708). The present invention is applicable to all the foregoing. It is especially applicable to cosmetic and personal care formulations in which the physical appearance of the product may be a major factor in promoting sales, for example, to shampoos, body lotions, shower gels or hair creams, It may also be applied to pharmaceutical preparations such as, drug delivery systems, and to flavourings and other concentrates for the food industry and to toothpastes.

## FLOCCULATION

A problem with the two phase structured surfactant systems, and especially spherulitic systems, is flocculation of the dispersed surfactant structures. This tends to occur at high surfactant and/or high electrolyte concentration. It can have the effect of making the composition very viscous and/or unstable with the dispersed surfactant separating from the aqueous phase.

Certain amphiphilic polymers have been found to act as deflocculants of structured surfactants. One type of deflocculant polymer exhibits cteniform (comb-shaped) architecture with a hydrophilic backbone and hydrophobic side chains or vice versa. A typical example is a random copolymer of acrylic acid and a fatty alkyl acrylate. Cteniform deflocculants have been described in a large number of patents, for example WO-A-9106622..

A more effective type of deflocculant has surfactant rather than cteniform architecture, with a hydrophilic polymer group attached at one end to a hydrophobic group. Such deflocculants are typically telomers formed by telomerising a hydrophilic monomer with a hydrophobic telogen. Examples of surfactant deflocculants include alkyl thiol polyacrylates and alkyl polyglycosides. Surfactant deflocculants are described in more details in EP O 623 670.

In a copending PCT patent application WO 01/00778 A1 filed on 22 June 2000 claiming priority from British patent application no. 9914673 we have described the use of small amounts (e.g. about 15% by weight of the composition) of carbohydrates such as sugars and alginates as deflocculants in structured surfactant compositions. The latter comprise surfactant, water and electrolyte in proportions adapted to form flocculated two-phase structured surfactant systems in the absence of the carbohydrate.

**THE PROBLEM**

Existing structured surfactant formulations are constrained by several limitations which have hitherto limited their application, especially in the areas of cosmetics and personal care. These include the following:-

1. Unless a substantial amount of electrolyte is present the choice of surfactant is limited to a fairly small range of relatively insoluble surfactants such as isopropyl alkyl benzene sulphonates. For many applications these are not the surfactants of choice from a performance point of view, and in some cases are totally inappropriate.

2. Spherulitic or dispersed lamellar structured surfactants are opaque. This limits the visual effects that can be achieved and may be perceived as less attractive than a clear system in some applications.

3. Expanded G phases are normally opalescent, have limited suspending power and are usually formed over narrow concentrations and/or temperature ranges which make them difficult to use in practice.

4. At high surfactant concentrations, e.g. above 25% by weight it is difficult to make stable structured systems without using expensive deflocculants and auxiliary stabilisers.

5. Most surfactant systems require preservatives to prevent microbial spoilage. However preservatives are expensive, ecologically undesirable, and may cause sensitivity problems for some users.

There is a need, especially in the personal care field, for a suspending system that is clear, transparent and mobile. There is a need for a system which contains high levels of surfactant but which does not require expensive deflocculants. There is a need for a system that contains relatively soluble surfactants but which does not require the presence of electrolyte as a structurant. There is a need for a cleaning composition which does not require added preservatives.

**The Solution**

We have now discovered that formulations meeting some or all of the above needs, may be obtained by using water soluble carbohydrate selected from mono and disaccharidc sugars, gluconic acid, mannic acid, ascorbic acid, sorbitol, mannitol and inositol to impart structure to the surfactant system, instead of or in addition to the electrolytes used hitherto. A component of the structured surfactant system which is used to impart structure to the surfactant will be referred to herein as a "structurant".

**The Invention**

Our invention provides the use of water soluble carbohydrates selected from mono and di-saccharide sugars, gluconic acid, mannic acid, ascorbic acid, sorbitol, mannitol and inositol as structurants in structured surfactant systems.

According to a second embodiment our invention provides a structured surfactant system having suspending properties which comprises from 1 to 60 weight percent of a surfactant, more than 20 weight percent of water and a structurant which comprises a water soluble carbohydrate which is present in the system in an amount of at least 25 weight percent by weight of the mixture of water, surfactant and carbohydrate and which is selected from mono and di-saccharide sugars, gluconic acid, mannic acid, ascorbic acid, sorbitol, mannitol and inositol. The structured system is an expanded G-phase system having a lamellar repeat spacing greater than 8nm, e.g. greater than 15nm. The composition, in the absence of suspended matter, is preferably clear and transparent. The composition further optionally comprises, if required in order to locate a sugar-structured phase, up to 30 weight percent of a co-structurant wherein the proportion of co-structurant is less than the proportion of carbohydrate.

The structured surfactant system may have a structural feature with a repeat spacing of 20 to 50nm.

According to a third embodiment the invention provides a composition comprising a structured surfactant system of the invention as specified above and suspended particles. The particles may be solid, liquid or gaseous and are either insoluble in the composition or present in excess of their solubility.

The invention also provides a packaged fluid surfactant-containing product, comprising an at least partially transparent container, and therein a stable structured surfactant of the invention as specified above, comprising a continuous phase and a dispersed phase and having a variegated appearance caused by the inclusion in localised portions of said structured surfactant of a dye or pigment which is insoluble in said continuous phase and present (A) as particles suspended in said continuous phase and having a particle size sufficiently small to be able to give said portions a substantially homogenous appearance which is visually distinct from other portions of said structured surfactant in said container, and/or (B) dissolved in or absorbed on said dispersed phase.

## THE STRUCTURED SYSTEM

The term "structured system" as used herein means a pourable composition comprising water, surfactant, dissolved carbohydrate and any other dissolved matter, including any costructurants, which together form a mesophase, or a dispersion of a mesophase in a continuous aqueous medium, and which has the ability to immobilise suspended particles while the system is at rest, to form a pourable suspension.

The aqueous structured systems formed by the interaction of surfactants with carbohydrates include systems which are believed to be in the form of an expanded G-phase. In particular they include novel systems having a much wider repeat spacing than the typical electrolyte-structured expanded G-phases described in EP O 530 708. The systems of the present invention comprise structures which typically show a repeat spacing between 20 and 50nm which is approximately double the repeat spacings measured for electrolyte-structured expanded G-phase, and approximately four times the typical repeat spacing in a conventional binary surfactant/water G-phase. The following discussion is based on the assumption that the structure is lamellar. We do not, however, intend to exclude the possibility that the system may comprise non-lamellar components.

Surprisingly, despite the apparently high lamellar spacing of the G-phases of the present invention, they are generally robust with good suspending power and good temperature stability. Typically the viscosity increases slightly with increasing temperature and the systems are often stable up to 70°C or higher.

The systems when fully deaerated and free from suspended fine insoluble particles are generally obtainable in a substantially clear and transparent form in marked contrast to other structured surfactant systems. This can typically be achieved by vigorous centrifugal deaeration and/or by gentle heating at, e.g. 60 to 80°C.

If the amount of surfactant or of structurant is not sufficiently high, or the ratio of electrolyte to carbohydrate is too high, the structured system of the invention will be obtained as an opaque two phase system which may be spherulitic or comprise dispersed G-pbase or batonettes.

## PROPORTIONS

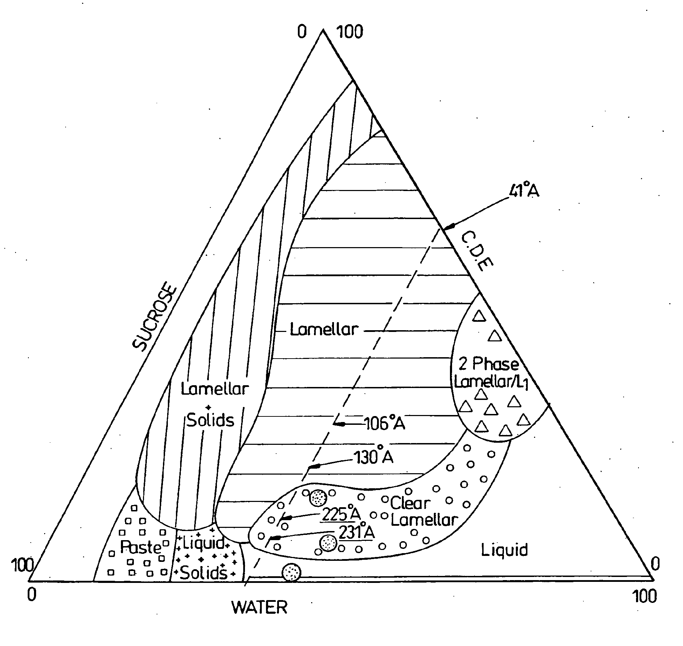

The proportions vary depending on the nature of the surfactant and of the carbohydrate. Figure 1 shows, in schematic form, a typical phase diagram for the system coconut diethanolamide, sucrose and water. The area marked "clear lamellar" represents the clear, pourable structured suspending system. The following typical proportions are expressed by weight of the total structured system i.e. comprising the water, surfactant, structurant and any other dissolved matter but excluding any suspended solids or water-immiscible liquids.

The surfactant is present in an amount of from 1 to 60 weight percent, generally the surfactant is present in an amount of at least 2%, e.g. at least 5% especially more than 10%, by weight of the system but preferably less than 50%, especially less than 40% more especially less than 30%. A convenient range is 3 to 25% especially 4 to 12%.

Carbohydrate structurants are usually required in substantially higher proportions than would be required for an electrolyte structurant. The carbohydrate is present in a proportion of at least 25%-e.g. at least 30% and usually more than 40% by weight. Concentrations greater than 65% are usually avoided. Typically the carbohydrate is less than 60%, usually less than 55% by weight of the composition. When electrolyte is present the carbohydrate may be present in substantially lower concentrations as a costructurant. Such systems may be spherulitic but in the presence of more than about 10% sugar, do not tend to flocculate. The systems of the invention require the presence of the carbohydrate in order to form a structured suspending system. Typically the less soluble the surfactant, the less carbohydrate is required.

The proportion of water is greater than 20% by weight, more commonly greater than 30%, typically greater than 40% of the system, but is preferably less than 65% usually less than 60%, e.g. less than 55%.

One way of preparing suspending systems according to the invention is to prepare a G or M phase aqueous surfactant and add sugar until the system clears. The G or M phases are located using conventional means, as described for example in GB 2 013 235.

Suspending power may be quickly checked by shaking air into the sample and noting whether the bubbles remain suspended. Confirmation that the system is a true structured system and not merely a slowly separating system may be obtained by allowing the sample to stand overnight at 50 or 60°C. If the dispersed phase has not separated out in that time, the system may be assumed to be structured. It is generally found that mixtures of two G-phase systems according to the invention also form G-phases according, to the invention.

If in any case difficulty is encountered locating a sugar-structured phase according to the invention, it is usually possible for resolve by adding a minor proportion based on the weight of sugar of a co-structurant has discussed below.

## THE CARBOHYDRATE

The preferred carbohydrates are mono and disaccharide sugars such as sucrose, glucose or fructose. The term "carbohydrate" as used here also includes the water soluble non-surfactant derivatives of carbohydrates gluconic acid, mannic acid and ascorbic acid and the reduced sugars sorbitol, mannitol and inositol. The levels of carbohydrate are preferably sufficiently high to inhibit microbiological growth in the medium and preferably sufficient to act as an effective biodegradable, non-allergenic preservative for the composition, thereby obviating the need for less environmentally friendly additives.

## CO-STRUCTURANT

Some surfactants, especially the more water soluble surfactants such as alkyl ether sulphates form the clear lamellar phase more readily in the presence of a costructurant. The co-structurant is preferably an electrolyte. Any water soluble salt which tends to lower the solubility of surfactant in water may be used, such as sodium tripolyphosphate, sodium carbonate, sodium citrate, sodium chloride or the corresponding potassium or ammonium salts. Alkalis such as sodium or potassium hydroxide may also be used. Other structurants include polar water-immiscible solvents such as phenolethoxy ether or a terpene water soluble mono and dihydroxy alcohols and ether alcohols such as glycerol, propylene glycol, ethylene glycol monomethyl ether and diethylene glycol monomethyl ether.

The constructurant, if required, may in principal, be present in concentrations up to 30%, but is preferably less than 20% e.g. 0-1 to 15% by weight Often traces of costructurants e.g. 0.1 to 3%, typically 0.5 to 2.5% by weight based on the system are sufficient, although higher concentrations can be present. For example in some perfumed systems, the solvent in the perfume may be sufficient to provide any desired co-structuring effect. The proportion of costructurant is less than the proportion of carbohydrate, preferably less than half the proportion of carbohydrate, e.g. less than one quarter the proportion of carbohydrate. Large amounts of electrolyte are generally undesirable because they inhibit the formation of clear phases.

Generally the costructurant is present in proportions insufficient to form a stable structured system in the absence of the carbohydrate.

## THE SURFACTANT

The surfactant preferably comprises non-ionic surfactants such as $C_{8-25}$ alkyl mono or diethenanolamides or 1 to 50 mole ethoxylates such as $C_{8-25}$ alcohol or fatty acid ethoxylates, alkyl amine ethoxylates, or glyceryl or sorbitan ester ethoxylates, or polyoxypropylene oxyethylene block copolymers. Ethoxylates typically contain from 2 to 40 eg. 3 to 30 especially 5 to 15 oxyethylene groups. Other non-ionic surfactants include alkyl polyglycosides, sugar esters or amine oxides. The non-ionic surfactants typically have a HLB of from 5 to 16, e.g. 6 to 15, especially 8 to 14, e.g. 10 to 12. However surfactants with HLB as low as 1 may be used.

The surfactant may optionally be or comprise an anionic surfactant such as an ether sulphate, an alkyl benzene sulphonate, an alkyl sulphate, alkane sulphonate, olefin sulphonate, sulphosuccinate, sulphosuccinamate, soap, sarcosinate, tauride, isethionate, alkyl phosphate, or alkyl ether carboxylate. In each case the surfactant comprises an 8 to 25 carbon alkyl group or alkenyl group or polypropyleneoxy group. Alkyl or alkenyl groups may be straight or branched chain, primary or secondary and preferably have from 10 to 20 eg. 12 to 14 carbon atoms. Ether groups may comprise glyceryl groups and/or 1 to 20 mol polyoxyethylene groups e.g. 2 to 10 mole. The anionic group is usually a sulphate or sulphonate group, but may also be for example, a phosphate, phosphonate or carboxylate group.

The counter ion of the anionic surfactant is usually sodium but may also be potassium, lithium, ammonium or, calcium or other alkali metal or alkaline earth metal.

The surfactant may be or may comprise an amphoteric surfactant such as betaine, sulphobetaine or phosphobetaine. Examples include fatty alkyl dimethyl betaines, alkyl amidopropyl betaines and immidazoline betaines.

The surfactant may, alternatively be or comprise a cationic surfactant such as a $C_{8-25}$ straight or branched alkyl or alkenyl or alkylphenyl tri $C_{1-4}$ alkyl or hydroxyalkyl ammonium salt, or di $C_{1-4}$ alkyl benzyl ammonium salt, or an $C_{8-20}$ alkyl or alkenyl amido amine.

## ELECTROLYTE

The presence of electrolyte is not normally required for structuring but is generally tolerated if required for other purposes. We particularly prefer electrolyte-free or low electrolyte (e.g. 1 to 5% by weight) compositions for personal care applications or where clear formulations are required but can tolerate much higher levels, e.g. up to 20% or more if required. For example industrial cleaning formulations may require high levels of alkali such as sodium hydroxide, carbonate or silicate. The presence of builders such as citrate, potassium pyrophosphate, or sodium tripolyphosphate may also be tolerated. Electrolyte may contribute to the structuring of the composition, and may be desirable as a costructurant when very water soluble surfactants or surfactants of high HLB are used.

## SUSPENDED MATTER

The composition may contain suspended solid, liquid or gaseous particles. For instance the composition may contain suspended oil droplets. The oil is preferably a mineral oil (e.g. a low molecular weight petroleum oil) or a fatty glyceride or other ester such as lauryl acetate, a terpene oil such as limonene or a silicone oil. Mixtures of oils may be used. Particularly preferred are vegetable oils such as coconut, evening primrose, groundnut, meadow foam, apricot kernel, peach kernel, avocado, jojoba and olive oil. Oil soluble cosmetic or topical pharmaceutical ingredients may be dissolved in the oil including antiseptics, styptics, antidandruff agents such as zinc omadine (zinc pyrithione) and selenium disulphide, proteins, emollients such as lanolin, isopropyl myristate, glyceryl isosterate or propylene glycol distearate, dyes, perfumes and waxes. Water insoluble particulate solids may be suspended including exfoliants such as talc, clays, polymer beads, sawdust, silica, seeds, ground nutshells and dicalcium phosphate, pearlisers such as mica or glycerol or ethylene glycol distearate, glitter additives and sunscreens such as titanium dioxide. Porous particles (so called micro-sponges) containing absorbed active ingredients or gelatin or other microcapsules may also be suspended. Other active ingredients which may be suspended include insect repellents and topical pharmaceutical preparations, e.g. preparations for treatment of acne, fungicides for athlete's foot or ringworm or antiseptics or antihistamines. Pigments, such as the iron oxides, may also be added.

The structured suspending systems of the invention may be used to suspend builders such as zeolite or sodium tripolyphosphate, agricultural and horticultural pesticides, biocides for water treatment, cuttings or shale in drilling muds, antifoams, explosives, gums such as gum bcnzoin, guan acacia, gum tragacanth xanthan and guar gum, enzymes, flavouring and vitamin concentrates, calcium phosphate for toothpaste, pharmaceuticals, and machinery and cutting abrasives such as emery or diamond powder.

The composition may contain liquefied propellant gas dispersed in order to provide foams such as shaving foam, on release from a pressurised pack.

## PEARLISING

The compositions of the invention are particularly useful for suspending pearlising agents. Pearlisers are required as concentrates for incorporation into liquid formulations such as shampoos and toiletries to import a nacreous iridescence which is attractive to consumers, and can mask inhomogeneities in the formulations.

Pearlisers typically comprise small, thin, transparent platelet crystals which can be suspended in a parallel configuration. When so suspended light falling on the crystals undergoes complex multiple reflections within the substrate similar to those which occur in a pearl and giving rise to similar optical interference effects.

Natural pearls comprise alternate layers of calcium carbonate and protein. Artificial pearlisers which can be suspended according to the invention include guanine/hypoxanthine crystals extracted from fish scales, mica, various salts of lead, zinc. mercury and bismuth (e.g. bismuth oxychloride), titanium oxide and various fatty acid derivatives such as magnesium stearate, coconut monoethanolamide, ethylene glycol distearate and ethylene glycol monostearate. Fish scale extracts are too expensive and the inorganic pearlisers are either too toxic for general use in toiletries e.g. lead, mercury, or relatively ineffective e.g. bismuth. The fatty acid derivatives are therefore now the most widely used pearlisers. In addition to the chemical nature and physical form of the pearliser the manner in which it is suspended has an important effect on its visual impact.

Difficulty is sometimes encountered obtaining the desired effect when incorporating pearlisers into aqueous formulations.

Conventional fatty acid derived pearlisers are supplied as solids which are usually added to a heated formulation above their melting point and recrystallised in situ. The conditions of crystallisation and especially the amount and nature of the agitation applied must be carefully controlled in order to obtain an acceptable result. This makes it difficult to obtain consistent effects and renders solid pearlisers inconvenient to use.

Attempts have been made to prepare liquid concentrates or suspensions which can be added directly to shampoo formulations without heating. While more convenient for the user, such concentrates face the manufacturer with problems of obtaining a high and consistent pearl effect, similar to those which confront the user of conventional solid pearlisers. Difficulty is also encountered in maintaining the particles in stable suspension and preventing sedimentation.

We have now discovered that carbohydrate structured phases of the invention have the capacity to form stable suspensions of pearlisers.

The pearliser may be dispersed in the aqueous structure surfactant system e.g. by gently stirring, but in the case of the fatty acid derivatives are preferably prepared in situ by heating above their melting point, e.g. temperatures between 65 and 80°C, dispersing the liquid pearliser in the structured surfactant system, preferably with sufficient stirring to form droplets of from 0.5 to 20 microns, e.g. 1 to 10 microns, and cooling to ambient temperature. Preferably cooling is relatively slow e.g. the mixture is allowed to cool naturally. The amount of pearliser can be varied considerably, the main constraint on the upper limit being the viscosity.

The amount of pearliser should not be so high as to render the product unpourable, or unacceptably viscous. We prefer on economic grounds that the pearliser is present in amounts greater than suspending surfactant. Generally pearliser may be present in amounts ranging from 5% up to about 50% e.g. 10 to 45% of the total weight of the mixture.

## OTHER INGREDIENTS

The composition may contain minor amounts of other ingredients such as dyes, perfumes, soil suspending agents or optical brighteners. Solvents such as ethanol or isopropyl alcohol ethylene glycol, isopropylene glycol, glycerol or water miscible glycol ethers such as ethylene glycol monomethyl ether, diethylene glycol monomethyl ether or polyethylene glycol, and hydrotypes such as $C_{1-6}$ alkyl benzene sulphonates or urea may be required for special applications, e.g. as perfume enhancers, but if not so required are generally undesirable and are preferably absent but may be tolerated in small amounts, preferably less than 10%, e.g. less than 5%, most preferably less than 2%.

## OPTICAL VARIEGATION

The present invention is particularly adapted to providing optically variegated fluid surfactant compositions.

Many fluid surfactant - containing products are purchased by the consumer on the basis of factors which include the appearance of the product. Detergents, shampoos, toiletries, soaps and other surfactant-based consumer products often depend upon appearance and packaging for at least part of their consumer appeal. Striped toothpaste, marbled soap and blue speckled detergent powder are well known examples of products whose successful promotion was based on a characteristically variegated appearance.

However it is not, on the face of it, possible to produce any kind of lasting variegation in an otherwise homogenous, pourable, liquid formulation.

It is known to combine two or more immiscible liquids of different density and colour to form a product which segregates into horizontal bands.

The visual effect achievable by this method is limited and the product has the practical disadvantage that the essential functional components are not evenly distributed between the different bands so that the product performs inconsistently if it is not vigorously agitated immediately prior to use.

Structured surfactant s may be used to suspend coloured granules, to produce a speckled effect.

We have now further discovered that where two or more portions of a structured surfactant such as those according to the present invention are separately coloured by including in at least one of said proportions a pigment, which is insoluble in said continuous phase or a dye which is insoluble in said continuous phase and soluble in or absorbable on any dispersed phase and said proportions are charged to a transparent container in such a way as to produce a variegated appearance, little or no migration of pigment or dye through the composition is observed in the undisturbed sample even after prolonged standing. The variegation thus remains stable to a remarkable degree. Furthermore, provided the container is substantially full, even the agitation caused by normal handling during distribution does not significantly affect the variegated appearance of the product. Yet the product may be, to all appearances, a thin, mobile liquid.

According to a further embodiment our invention therefore provides a packaged fluid surfactant-containing product, comprising an at least partially transparent container and therein a stable structured surfactant according to the invention, as specifies above, comprising a continuous phase and a dispersed phase and having a variegated appearance caused by the inclusion in localized portions of said structured surfactant of a dye or pigment which is insoluble in said continuous phase and present (A) as particles suspended in said continuous phase and having a particle size sufficiently small to be able to give said portions a substantially homogenous appearance which is visually distinct form other portions of said structured surfactant in said container, and/or (B) dissolved in or absorbed on said dispersed phase.

The effects obtainable can be extremely varied. Depending upon how the different visually distinct portions of the structured surfactant are charged to the container it is possible to obtain horizontal or vertical stripes, vertical segments, marbling, bands, whorls or numerous other decorative effects.

Any pattern or optical effect which can be instantaneously obtained by charging visually distinct liquids to a transparent container can be rendered substantially permanent, at least until the product is poured from the container, by using the structured surfactants as said liquids. With suitable filling techniques, it is even possible to produce readable characters so that liquid products may be marked with Trademarks, logos or similar devices.

The pigment or dye may for example be, or comprise a water insoluble pigment, having a particle size preferably less than 150 microns especially less than 100 microns, most preferably less than 50 microns e.g. 0.1 to 20 microns.

The proportion of pigment required is generally small. Any pigmented portion normally only requires from 0.001 to 1% by weight of pigment to produce a sufficient effect e.g. 0.01 to 0.5% more usually 0.02 to 0.1%. The precise amount will depend on the choice of pigment and the intensity of colour required.

The pigment may be a white pigment (e.g. titanium dioxide) a black pigment (e.g. carbon black), coloured pigment such as any water insoluble pigment hitherto used in cosmetics or detergents, or a pearlising agent such as mica.

Typically the structured surfactant has an aqueous continuous phase and the pigment or dye is water insoluble. Water insoluble dyes typically dissolve in or are absorbed on a dispersed surfactant phase.

We prefer that, apart from the pigment or dye, the differently coloured portions of the structured surfactant product of our invention should have essentially the same composition. This assists maintaining the stability of the product and ensures uniform performance. However it is possible e.g. where substantially water insoluble active ingredients other than pigment are suspended in the product or dissolved or otherwise contained in a suspended oil phase, to include such ingredients in only some portions of the product. This may be useful in segregating mutually incompatible components and may permit promotional claims that the coloured portions are associated with a specific beneficial effect.

The method of filling the container determines the effects produced. For example horizontal stripes can be obtained by running a single bladed stirrer gently in a container partly filled with one coloured structured surfactant and injecting a contrasting colour through a syringe at the level of the stirrer. Progressively raising the stirrer and repeating the process produces a plurality of horizontal stripes. Injecting the contrasting liquid while drawing the syringe up the side of the container produces a vertical stripe. Inserting a partition into the container, and filling different portions on either side, before withdrawing the partition, produces contrasting vertical halves. A multiple partition permits vertical segments. Alternatively vertical effects may be produced by inserting two or more tubes into the container and gradually withdrawing the tubes while charging the container with different coloured portions, each at the same rate.

Partially stirring two contrasting portions together before filing the container gives an attractive marbled effect. The foregoing are merely an indication of some of the different filling techniques and associated visual effects possible according to the invention. Numerous other possibilities will be apparent to those skilled in the art.

The container may be any jar, bottle, tube, sachet or other conventional container for surfactant based products. It may typically be of glass or plastic or other transparent material. It may be coloured but is preferably at least partly clear to enable the decorative contents to be easily seen. It is possible to use deformable containers such as squeeze tubes or sachets, provided that they are sufficiently filled to give them a degree of rigidity enough to avoid loss of variegation on normal handling prior to use, but it is preferred to use rigid or at least substantially non-deformable materials.

The invention will be illustrated by the following examples in which all proportions are based on weight percentages of active ingredient based on the total weight of the composition, unless stated to the contrary.

## Examples 1 to 4

The following formulations were prepared:-

|                                        | 1       | 2       | 3       | 4       |
|----------------------------------------|---------|---------|---------|---------|
| $C_{12-14}$ 3 mole ether sulphate      | 10      | 12.5    | 15      | 17.5    |
| Sucrose                                | 46      | 46      | 46      | 46      |
| Trisodium citrate                      | 2       | 2       | 2       | 2       |
| Perfume                                | 5       | 5       | 5       | 5       |
| Water                                  | balance | balance | balance | balance |

Ali four formulations were clear or slightly hazy, mobile structured liquids with good suspending properties. Suspensions of talc, mineral oil, pigment, small beads and plastic novelty items were prepared. All were stable after prolonged storage.

Each of the examples 1 to 4 was re-prepared (a) without the perfume (b) without the citrate and (c) without perfume or citrate. No suspending power was exhibited by any of the eight samples of preparations (a) and (c). The samples of preparation (b) all exhibited similar suspending power to the original examples. On heating the samples to 70°C and subsequent cooling a clear transparent composition was obtained.

A clear sample of Example 1(b) and a sample containing a red pigment in suspension were slowly poured into a sample jar in a series of alternating additions. The effect was to produce a sequence of horizontal stripes. When the bottle was filled the stripes retained their integrity and showed no signs of blurring or diffusion after one year storage including intermittent periods of gentle shaking and six months weeks stored on its side.

## Example 5

A schematic phase diagram was prepared for the system coconut diethanelamide/sucrose/water and is reproduced as fig. 1 of the drawings. The area marked "clear lamellar" represents transparent expanded G-phases having a suspending power according to the invention and the area marked "lamellar" comprises opaque expanded lamellar suspending systems according to the invention. The phase boundaries illustrated were not all precisely determined.

## Example 6

A sample was prepared comprising 10% coconut diethanolamide, 35% water and 55% sucrose. The product was a clear attenuated G-phase with good suspending power.

## Example 7

A composition comprising 10% $C_{12-14}$ alkyl 2 mole ethoxysulphate, 33% water, 50% sucrose, 5% ethanol based perfume, 2% sodium citrate gave a clear, attenuated G-phase with good suspending powers.

## Example 8 (Shampoo formulation)

4 parts by weight of the composition of Example 7 and 1 part of the composition of Example 6 were mixed together to form a clear composition of the invention with good suspending powers and good performance as a skin cleanser and shampoo.

## Example 9 (laundry detergent formulation)

|  | % Active ingredient by weight |
|---|---|
| $C_{12-14}$ linear alkyl benzene sulphonate | 6.6 |
| Triethanolamine lauryl sulphate | 1.65 |
| $C_{12-14}$ alkyl 3 mole ethoxylate | 1.6 |
| Sucrose | 55.0 |
| Sodium diethlenetriamine pentakis (methylenephosphonate) | 0.55 |
| Water | balance |

The product was a hazy, readily pourable liquid with good suspending power.

## Example 10 (Pearl concentrate)

A pearl concentrate was obtained by heating a formulation comprising 54% by weight sucrose, 10% by weight coconut di-ethanolamide, 10% by weight ethylene glycol distearate and 26% by weight water to 70°C and cooling.
A spontaneously pearly suspension was obtained.

## Example 11

10% by weight $C_{12-14}$ alkyl six mole ethoxylate (HLB = 10), 54% by weight sucrose and 36% by weight water, were mixed and warmed to 70°C.
The cooled product was a clear transparent, pourable system with good suspending properties.

## Example 12

|  | % w/w |
|---|---|
| succrose | 40.0 |

(continued)

| | % w/w |
|---|---|
| perfume | 2.0 |
| sodium $C_{12-14}$ alkyl 3 mole ethoxy sulphate (70%) | 8.0 |
| coconut diethanolamide | 2.0 |
| sodium chloride | 5.0 |
| water | balance |

The above formulation provided a clear, transparent, pourable fluid. Whorls of three different coloured pigments were introduced into this formulation with a syringe. After three months no diffusion of the pigment was observable.

## Example 13

| | % w/w |
|---|---|
| succrose | 40.0 |
| perfume | 2.0 |
| trisodium citrate dihydrate | 2.0 |
| sodium $C_{12-14}$ alkyl 3 mole ethoxy sulphate (70% by wt active) | 8.8 |
| glycamate | 5.06 |
| coconut monoethanolamide | 1.1 |
| coconut amido propyl betaine | 4.18 |
| sodium $C_{8-10}$ alkyl polyglycoside dp 1.6 (65%) | 6.2 |
| sodium chloride | 0.66 |
| sodium ethylene diamine tetraacetate | 0.05 |
| water | balance |

The composition was a pourable, clear, transparent fluid with suspending properties. A plurality of coloured, polystynene beads (1mm diametre) were dispersed in the composition. The suspension remained stable after three months.

## Example 14

| | % w/w |
|---|---|
| sodium $C_{12-14}$ alkyl (3 mole ethoxy sulphate) | 10.0 |
| coconut diethanolamide | 2.5. |
| fructose | 50.0 |
| water | balance |

The above composition was a clear isotropic $L_1$ micellar solution which was unsaturated and had no suspending power. Addition of 6% by weight sodium chloride, gave a stable, easily pourable fluid, composition which after shaking was capable of suspending air bubbles. The aerated composition was stood overnight at 50°C. The aged composition was clear and transparent and maintained the air bubbles in a stable suspension. Equivalent compositions with 2 and 4% respectively of sodium chloride were not able to suspend bubbles under the foregoing conditions.

## Example 15

|  | % w/w |
|---|---|
| sodium $C_{12-14}$ alkyl -3 mole ethoxy sulphate | 8.0 A.I. |
| coconut diethanolamide + 10% by wt glycerol | 2.0 A.I. |
| fructose | 40.0 |
| water | balance |

The above composition was not capable of maintaining particles in suspension. Addition of incremental amounts of sodium chloride gave the following results:-

| wt % sodium chloride | |
|---|---|
| 2 | L I phase bubbles rise |
| 4 | L1 phase bubbles rise |
| 6 | clear suspending phase. Bubbles suspended after ageing at 50°C overnight. |

**Claims**

1. The use of water soluble carbohydrates selected from mono and disaccharide sugars, gluconic acid, mannic acid, ascorbic acid, sorbitol, mannitol and inositol as structurants in structured surfactant systems.

2. A structured surfactant system having suspending properties which is an expanded G-phase system having a lamellar repeat spacing greater than 8nm, and which comprises:

   from 1 to 60 weight percent of a surfactant,
   more than 20 weight percent of water, and
   a structurant, wherein said structurant comprises a water soluble carbohydrate, which is present in the system in an amount of at least 25 weight percent by weight of the mixture of water, surfactant and carbohydrate, and which is selected from mono and di-saccharide sugars, gluconic acid, mannic acid, ascorbic acid, sorbitol, mannitol and inositol,
   and optionally, if required in order to locate a sugar-structured phase, up to 30 weight percent of a co-structurant, wherein the proportion of co-structurant is less than the proportion of carbohydrate.

3. A system according to claim 2 wherein said G-phase has a lamellar repeat spacing greater than 15nm.

4. A system according to claim 3 which is transparent.

5. A. structured surfactant system according to claim 2, having a structural feature with a repeat spacing of from 20 to 50nm.

6. A system according to any one of claims 2 to 5 containing 30 to 60% by weight water.

7. A system according to any one of claims 2 to 6 comprising 0.1. to 15% by weight of co-structurant.

8. A system according to any one of claims 2 to 7 wherein the costructurant is an electrolyte or a polar water-immiscible solvent.

9. An aqueous suspension comprising a system according to any of claims 2 to 8 and particles of solid, liquid or gas stably suspended therein.

10. A composition according to claim 9 wherein said particles comprise a builder.

11. A composition according to either of claims 9 or 10 wherein said particles comprise an abrasive.

12. A composition according to any of claims 9 to 11 wherein said particles comprise a pesticide.

**13.** A composition According to any of claims 9 to 12 wherein said particles comprise an oil.

**14.** A composition according to any of claims 9 to 13 wherein said particles comprise a pigment.

**15.** A composition according to claim 14 having a plurality of differently pigmented zones producing a variegated visual effect.

**16.** A packaged fluid surfactant-containing product, comprising an at least partially transparent container, and therein a stable structured surfactant according to claim 2 comprising a continuous phase and a dispersed phase and having a variegated appearance caused by the inclusion in localised portions of said structured surfactant of a dye or pigment which is insoluble in said continuous phase and present (A) as particles suspended in said continuous phase and having a particle size sufficiently small to be able to give said portions a substantially homogenous appearance which is visually distinct from other portions of said structured surfactant in said container, and/or (B) dissolved in or absorbed on said dispersed phase.

**Patentansprüche**

**1.** Verwendung wasserlöslicher Kohlenhydrate ausgewählt aus Mono- und Disaccharid-Zuckern, Gluconsäure, Mannonsäure, Ascorbinsäure, Sorbitol, Mannitol und Inositol als strukturgebende Substanzen in strukturierten Tensid-Systemen.

**2.** Strukturiertes Tensid-System mit suspendierenden Eigenschaften, das ein erweitertes G-Phasen-System darstellt, welches einen Lamellenwiederholabstand größer als 8nm besitzt, und das enthält:

> 1-60 Gewichtsprozent eines Tensides
> mehr als 20 Gewichtsprozent Wasser und
> eine strukturgebende Substanz, wobei diese strukturgebende Substanz ein wasserlösliches Kohlenhydrat enthält, welches im System in einer Menge von wenigstens 25 Gewichtsprozent bezogen auf das Gewicht der Mischung Wasser/Tensid/Kohlenhydrat vorhanden ist und welches ausgewählt ist aus Mono- und Disaccharid-Zuckern, Gluconsäure, Mannonsäure, Ascorbinsäure, Sorbitol, Mannitol und Inositol
> und optional, falls erforderlich um eine zucker-strukturierte Phase unterzubringen, bis zu 30 Gewichtsprozent einer weiteren strukturgebenden Substanz, wobei der Anteil der weiteren strukturgebenden Substanz geringer ist als der Anteil des Kohlenhydrats.

**3.** System gemäß Anspruch 2, worin die G-Phase einen Lamellenwiederholabstand größer als 15nm besitzt.

**4.** System gemäß Anspruch 3, das transparent ist.

**5.** Strukturiertes Tensid-System gemäß Anspruch 2, das ein Strukturmerkmal mit einem Wiederholabstand von 20 bis 50 nm besitzt.

**6.** System gemäß jedem der Ansprüche 2 bis 5, das 30 bis 50 Gewichtsprozent Wasser enthält.

**7.** System gemäß irgendeinem der Ansprüche 2 bis 6, das 0,1 bis 15 Gewichtsprozent der weiteren strukturgebenden Substanz enthält.

**8.** System gemäß irgendeinem der Ansprüche 2 bis 7, worin die weitere strukturgebende Substanz ein Elektrolyt ist oder ein polares, mit Wasser nicht mischbares Lösungsmittel.

**9.** Wässrige Suspension, die ein System gemäß irgendeinem der Ansprüche 2 bis 8 enthält und darin stabil suspendierte feste, flüssige oder gasförmige Teilchen.

**10.** Zusammensetzung gemäß Anspruch 9, worin die besagten Partikel einen Träger enthalten.

**11.** Zusammensetzung gemäß irgendeinem der Ansprüche 9 oder 10, worin die besagten Partikel ein Schleifmittel enthalten.

**12.** Zusammensetzung gemäß irgendeinem der Ansprüche 9 bis 11, worin die besagten Partikel ein Pestizid enthalten.

**13.** Zusammensetzung gemäß irgendeinem der Ansprüche 9 bis 12, worin die besagten Partikel ein Öl enthalten.

**14.** Zusammensetzung gemäß irgendeinem der Ansprüche 9 bis 13, worin die besagten Partikel ein Pigment enthalten.

**15.** Zusammensetzung gemäß Anspruch 14, die eine Vielzahl verschieden pigmentierter Zonen enthält, welche einen bunten Seheffekt erzeugen.

**16.** Abgepacktes fluides tensid-haltiges Produkt, das einen wenigstens teilweise durchsichtigen Behälter aufweist und darin ein stabiles strukturiertes Tensid gemäß Anspruch 2, welches eine kontinuierliche Phase und eine disperse Phase besitzt und welches ein bunter Erscheinungsbild hat, das durch den Einschluss in lokalisierte Abschnitte des strukturierten Tensids einer Farbe oder eine Pigments verursacht wird, welches unlöslich in der kontinuierlichen Phase ist und vorhanden ist (A) als suspendierte Teilchen in der kontinuierlichen mit einer Teilchengröße, die klein genug ist um den Abschnitten ein im wesentlichen homogenes Erscheinungsbild zu geben, welches sichtbar verschieden ist von anderen Abschnitten des strukturierten Tensids in dem Behälter und/oder
(B) gelöst in oder absorbiert an der dispersen Phase.

## Revendications

**1.** Utilisation de glucides hydrosolubles sélectionnés parmi les sucres mono et disaccharidiques, l'acide gluconique, l'acide mannique, l'acide ascorbique, le sorbitol, le mannitol et l'inositol en tant que structurants dans les systèmes tensioactifs structurés.

**2.** Système tensioactif structuré ayant des propriétés suspensives qui est un système de phase G expansée ayant un espacement de répétition lamellaire supérieur à 8 nm, et qui comprend :

de 1 à 60 pour cent en poids d'un tensioactif,
plus de 20 pour cent en poids d'eau, et
un tensioactif, dans lequel ledit structurant comprend un glucide hydrosoluble, qui est présent dans le système en une quantité d'au moins 25 pour cent en poids du mélange d'eau, de tensioactif et de glucide, et qui est sélectionné parmi les sucres mono et disaccharidiques, l'acide gluconique, l'acide mannique, l'acide ascorbique, le sorbitol, le mannitol et l'inositol,
et facultativement, si nécessaire afin de localiser la phase structurée de sucre, jusqu'à 30 pour cent en poids d'un co-structurant, dans lequel la proportion de co-structurant est inférieure à la proportion de glucide.

**3.** Système selon la revendication 2 dans lequel ladite phase G a un espacement de répétition lamellaire supérieur à 15 nm.

**4.** Système selon la revendication 3 qui est transparent.

**5.** Système tensioactif structuré selon la revendication 2, ayant une caractéristique structurelle avec un espacement de répétition allant de 20 à 50 nm.

**6.** Système selon l'une quelconque des revendications 2 à 5 contenant 30 à 60 % en poids d'eau.

**7.** Système selon l'une quelconque des revendications 2 à 6 comprenant 0,1 à 15 % en poids de co-structurant.

**8.** Système selon l'une quelconque des revendications 2 à 7 dans lequel le co-structurant est un électrolyte ou un solvant polaire immiscible dans l'eau.

**9.** Suspension aqueuse comprenant un système selon l'une quelconque des revendications 2 à 8 et des particules de solide, de liquide ou de gaz stablement suspendues dans celui-ci.

**10.** Composition selon la revendication 9 dans laquelle lesdites particules comprennent un adjuvant.

**11.** Composition selon l'une des revendications 9 ou 10 dans laquelle lesdites particules comprennent un abrasif.

**12.** Composition selon l'une quelconque des revendications 9 à 11 dans laquelle lesdites particules comprennent un pesticide.

**13.** Composition selon l'une quelconque des revendications 9 à 12 dans laquelle lesdites particules comprennent une huile.

**14.** Composition selon l'une quelconque des revendications 9 à 13 dans laquelle lesdites particules comprennent un pigment.

**15.** Composition selon la revendication 14 ayant une pluralité de zones différemment pigmentées produisant un effet visuel panaché.

**16.** Produit fluide emballé contenant un tensioactif, comprenant un récipient au moins partiellement transparent, et à l'intérieur de celui-ci un tensioactif structuré stable selon la revendication 2 comprenant une phase continue et une phase dispersée et ayant un aspect panaché provoqué par l'inclusion dans des portions localisées dudit tensioactif structuré d'un colorant ou pigment qui est insoluble dans ladite phase continue et présent (A) sous la forme de particules suspendues dans ladite phase continue et ayant une taille de particules suffisamment petite pour pouvoir donner auxdites portions un aspect substantiellement homogène qui est visuellement différent des autres portions dudit tensioactif structuré dans ledit récipient, et/ou (B) dissous dans ou absorbé sur ladite phase dispersée.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP O086614 A
- EP O151884 A
- EP O530708 A
- EP O414549 A
- EP O388239 A
- EP O498231 A
- EP O430602 A
- EP O472089 A
- WO 9106622 A
- EP O623670 A
- WO 0100778 A1
- GB 9914673 A
- GB 2013235 A